# EUROPEAN PATENT SPECIFICATION

(11) **EP 3 558 037 B1**
(45) Date of publication and mention of the grant of the patent: **30.12.2020**
(21) Application number: 17801695.2
(22) Date of filing: 17.11.2017
(51) Int. Cl.: A24F 47/00, A61M 15/06, B05B 11/00

(54) **REFILLING SYSTEM FOR AEROSOL INHALER**
NACHFÜLLSYSTEM FÜR AEROSOLINHALATOR
SYSTÈME DE RECHARGE POUR INHALATEUR AÉROSOL

(30) Priority: 23.12.2016 EP 16206774
(43) Date of publication of application: 30.10.2019
(73) Proprietor: JT International SA, 1202 Geneva (CH)
(72) Inventor: STALDER, Roland, 8055 Zurich (CH); WALDERN, Frederick, 8057 Zurich (CH); PAUL, Samuel John, Ballymena Antrim BT44 9EU (GB)
(74) Representative: Gill Jennings & Every LLP
(86) International application number: PCT/EP2017/079671
(87) International publication number: WO 2018/114163

(56) References cited:
- WO-A1-2004/092016
- WO-A1-2016/203167
- FR-A1- 2 695 111
- FR-A1- 2 802 447

## Description

The present invention relates to an assembly for refilling a tank in an aerosol inhaler.

Electronic cigarettes and other aerosol inhalers are becoming increasingly popular consumer products. In these products an aerosol forming substance is stored in a tank in liquid form. The tank typically has an outlet connected to a wicking element which supplies the aerosol forming substance to an atomiser. The atomiser includes a heating coil that vaporises the liquid aerosol forming substance. A battery is connected to the atomiser, which is typically operated by a button or an air pressure sensor. Air inlets are provided so that the user can draw air into the device through or past the atomiser. In use, a user activates the atomiser and inhales the aerosol that is generated using a mouthpiece.

A problem arises in how to refill the tank in these devices. Exemplary refilling assemblies are shown e.g. in WO 2004/092016 A1 or FR 2 802 447 A1. Conventional techniques have proven to be slow and inefficient. Some techniques also cause leakage of the aerosol forming substance, which is considered undesirable.

An object of the present invention is to overcome and mitigate some of these problems.

According to an aspect of the invention there is provided a refilling assembly for refilling a receiving reservoir in an aerosol inhaler with an aerosol forming substance, the system comprising: a supply reservoir to contain the aerosol forming substance; a supply conduit in fluid communication with the supply reservoir for supplying the aerosol forming substance to the receiving reservoir; a return conduit configured to receive fluid from the receiving reservoir when the aerosol forming substance is supplied to the receiving reservoir by the supply conduit; a connection member configured to provide a sealed connection between the receiving reservoir, the supply conduit and the return conduit; and a displaceable member operable to displace the aerosol forming substance from the supply reservoir, through the supply conduit, towards the receiving reservoir, characterised in that the refilling assembly further comprises a peristaltic pump which includes the displaceable member, wherein the peristaltic pump is configured to pump the aerosol forming substance from the supply reservoir towards the receiving reservoir.

In this way, a convenient arrangement is provided for refilling the receiving reservoir in the aerosol inhaler. The displaceable member can be operated to pump the aerosol forming substance from the supply reservoir to the receiving reservoir. This can allow control of the volume of the substance that is supplied and the rate of supply can also be controlled to allow quick filling, without negative effect on the aerosol inhaler.

The displaceable member is preferably movable from a first position towards a second position such that movement of the displaceable member operably increases fluid pressure in the supply reservoir. Such an increase in fluid pressure in the supply reservoir may drive the aerosol forming substance through the supply conduit towards the receiving reservoir. The displaceable member may be provided as a moveable component in a mechanical or electrical pump; in one example, the displaceable member may be provided as a deformable wall in the supply reservoir or a plunger which can be driven from the first position towards the second position.

The sealed connection between the receiving reservoir, the supply conduit and the return conduit may advantageously ensure that fluid displaced from the receiving reservoir is directed through the return conduit. This may allow easy dispensing of the aerosol forming substance through the supply conduit. In embodiments a hermetic seal may be provided.

Preferably the refilling assembly comprises a return reservoir in fluid communication with the return conduit, the return reservoir configured to receive fluid from the receiving reservoir. The return reservoir is preferably provided in fluid communication with the supply reservoir so that a closed circuit is provided. Fluid may be displaced from the receiving reservoir when the aerosol forming substance is supplied to it. The fluid displaced from the receiving reservoir may include, at least, air and/or the aerosol forming substance.

The supply reservoir and return reservoir may be arranged in a common housing, which may be a single handheld housing.

Preferably a sealed circuit for transmission of fluid is provided between the supply reservoir and the return reservoir, via the receiving reservoir, the sealed circuit including the supply reservoir, the supply conduit, the connection member, and the return conduit. The receiving reservoir may also be part of the sealed circuit, when connected. In embodiments the sealed circuit may be a hermetically sealed circuit.

The displaceable member may be end-user displaceable. Thus, an end-user may be able to displace the displaceable member, for example by gripping, pressing. The end-user may be able to displace the displaceable member with their hand, and specifically with one or more digits.

The displaceable member may comprise a deformable wall of the supply reservoir. The deformable wall may be flexible, and may be displaced in order to increase fluid pressure on the supply reservoir in order to drive the aerosol forming substance through the supply conduit towards the receiving reservoir. Preferably the fluid in the sealed circuit includes a gaseous component, which can be compressed easily, relative to a liquid component. In this way, the deformable wall of the supply reservoir can be displaced easily by the compression of the gaseous component in the sealed circuit. Following deformation of the wall of the supply reservoir it has been found that fluid pressures cause a re-expansion of the gaseous component so that the gas returns to its previous volume. Advantageously this can cause the deformable wall to return to its original position after it has been displaced. This can provide a desirable user experience as the deformable wall snaps back to its previous position, ready to be displaced again in another pump action.

In embodiments the return reservoir may comprise a deformable wall, which can act as the displaceable member. This can draw fluid from the receiving reservoir into the return reservoir, creating a negative pressure in the receiving reservoir which acts to draw aerosol forming substance into the receiving reservoir from the supply reservoir. Thus, it may be a matter of perspective as to whether the aerosol forming substance displaces fluid in the receiving reservoir, or whether the fluid received by the return reservoir displaces the aerosol forming substance in the supply reservoir. Both interpretations may be possible in a sealed circuit where fluid flows in the system to balance differences in pressure that arise.

The displaceable member may be displaceable away from a first position in order to displace the aerosol forming substance from the supply reservoir, through the supply conduit, towards the receiving reservoir, and the displaceable member may be biased towards the first position. In this way, the displaceable member can be encouraged to return to the first position, after it has been used to pump the aerosol forming substance from the supply reservoir towards the receiving reservoir. The biasing means may be pneumatic, based on pressure differences that arise in a closed circuit. The biasing means may be mechanical, and mechanical biasing means may be provided in addition to pneumatic biasing means.

In one arrangement mechanical biasing means may include a flexible wall in the supply reservoir or the return reservoir. In another arrangement the displaceable member may be a plunger rod which includes a spring-based biasing means for urging it towards the first position.

The displaceable member may be displaceable from a first position to a second position in order to displace a first volume of the aerosol forming substance which is substantially matched to a volume of the receiving reservoir. In embodiments the first volume of the aerosol forming substance may be within at least 70 or 80% of the volume of the receiving reservoir. In this way, a single operation of the displaceable member can dispense the first volume of aerosol forming substance in order to substantially fill the receiving reservoir. This can allow a user to fill the receiving reservoir in a single pump action. In embodiments the first volume may be around 1ml, which may be adequate to fill the receiving reservoir. The supply reservoir may be larger; perhaps around 2ml, which may enable around two refill operations. The return reservoir may have a volume of around 2ml.

In one arrangement the peristaltic pump is an electric pump. The electric pump is configured to pump the aerosol forming substance from the supply reservoir towards the receiving reservoir. The displaceable member may include a first engagement member in the peristaltic pump to provide a constriction in the supply conduit or the return conduit. The constriction in the relevant conduit can then pump the aerosol forming substance supply reservoir to the receiving reservoir, with displaced fluid from the receiving reservoir being received by the return reservoir, via the return conduit. The displaceable member may also include a second engagement member to provide a moveable constriction in the supply conduit or the return conduit for pumping of the fluid from the supply reservoir to the receiving reservoir.

In other embodiments the peristaltic pump may be actuated manually, pneumatically, or using another driving force, as would be known to a person skilled in the art.

A peristaltic pump can advantageously provide a very low overpressure during pumping. Thus, the aerosol forming substance can fill the receiving reservoir without also urging the aerosol forming substance out of the receiving reservoir. In embodiments the receiving reservoir may include an outlet including a wicking element for supplying the aerosol forming substance to a heater for use in an inhaler. It has been found that high pressure delivery of the aerosol forming substance can undesirably drive the substance through the outlet in the receiving reservoir and into the wicking element, causing leakage. This is advantageously avoided by the use of a peristaltic pump having a low operating pressure. A low operating pressure can be achieved by providing a supply conduit and a return conduit that pump fluid to the receiving reservoir and away from the receiving reservoir at equal rates.

In embodiments the first and second engagement members of the peristaltic pump may be positioned proximal to the outlet of the supply conduit and the inlet of the return conduit. This advantageously minimises the volume of the aerosol forming substance that is held in the supply and return conduits when the receiving reservoir is disconnected. This advantageously reduces leakage when the receiving reservoir is disconnected.

In embodiments the first and second engagement members may be arranged on a common body of the displaceable member. Thus, a single displacement of the body can cause pumping in the supply conduit and the return conduit simultaneously. The displacement of the common body may be rotational, and the first and second engagement members may be connected to the common body or formed integrally with it.

An outlet of the supply conduit may be arranged to point in a first direction and an inlet of the return conduit may be arranged to point in a second direction, wherein the first and second directions are different from one another. In this way, there is a reduced risk that fluid dispensed from the outlet can be received directly by the inlet. Instead, fluid from the outlet is advantageously received in the receiving reservoir and displaced gas is advantageously received in the inlet.

In one arrangement the inlet and outlet may be peripherally disposed about a common axis and the first and second directions are arranged on respective radially extending lines that are separated by 90 - 270° or 120 - 240°. In embodiments the first and second directions may be substantially opposite to one another.

The connection member may include a protrusion that can receive a storage portion, which comprises the receiving reservoir, the protrusion including the inlet and the outlet. In an alternative arrangement the connection member may include a recess, including the inlet and the outlet, and the storage portion may be received in the recess.

The supply reservoir may comprise the aerosol forming substance.

According to another aspect of the invention there is provided a system for refilling the receiving reservoir of an aerosol generation system with an aerosol forming substance, the system comprising: the refilling assembly as defined above; and a storage portion including the receiving reservoir adapted to be connected to the connection member. The storage portion preferably includes an atomiser for generation of aerosol from the aerosol forming substance.

The storage portion is preferably an aerosol inhaler such as an electronic cigarette system comprising an atomiser, a power supply, a flow path including an inlet, a mouthpiece which acts as an outlet, where the atomiser receives the aerosol forming substance from the receiving reservoir and supply aerosol to the flow path for a user to inhale.

The storage portion is preferably shaped in order to complement the connection member, with a protrusion or a recess in specific examples. The receiving reservoir preferably comprises a valve system which can be actuated when the storage portion is assembled to the connection member. The valve system can be operated in order to provide fluid communication between the receiving reservoir, the supply conduit and the return conduit. The valve system advantageously prevents leakage from the receiving reservoir after the connection member has been disassembled.

According to another aspect of the invention there is provided a use of the refilling assembly as defined above for refilling a receiving reservoir of an aerosol generation system with an aerosol forming substance.

According to yet another aspect of the invention there is provided a method of refilling a receiving reservoir of an aerosol generation system with an aerosol forming substance, the method comprising the steps of: providing a sealed connection between the receiving reservoir, a supply conduit and a return conduit; actuating a displaceable member to displace the aerosol forming substance from the supply reservoir, through the supply conduit, to the receiving reservoir; and receiving fluid from the receiving reservoir in the return conduit; characterised in that the method further comprises operating a peristaltic pump which includes the displaceable member, wherein the peristaltic pump is configured to pump the aerosol forming substance from the supply reservoir towards the receiving reservoir.

The method may also comprise transmitting the fluid received in the return conduit to the return reservoir.

Apparatus features may be provided as method features, and *vice*-versa.

Embodiments of the invention are now described, by way of example, with reference to the drawings, in which:
Figure 1 is a cross-sectional view of a refilling bottle in an embodiment of the present invention;
Figure 2 is a cross-sectional view of an electronic cigarette having a tank that can be refilled with a refilling bottle in an embodiment of the present invention;
Figures 3A-3E are diagrams showing a series of steps that can be undertaken in a refilling operation in an embodiment of the present invention;
Figure 4 is a cross-sectional view of a refilling bottle connected to an aerosol inhaler in another embodiment of the present invention;
Figures 5A-5D are diagrams showing a series of steps that can be undertaken to detach a refilling bottle from an electronic cigarette in an embodiment of the invention;
Figure 6 is a perspective view of a desktop refilling assembly in an embodiment of the invention;
Figure 7 is a cross-sectional view of the desktop refilling assembly shown in Figure 6;
Figure 8 is a connector for use with a desktop refilling assembly in an embodiment of the present invention;
Figure 9 is a cross-sectional view of an aerosol inhaler connected to a connection member for use with a desktop refilling assembly in an embodiment of the present invention; and
Figure 10 is a plan view of a peristaltic pump for use with a desktop refilling assembly in an embodiment of the present invention.

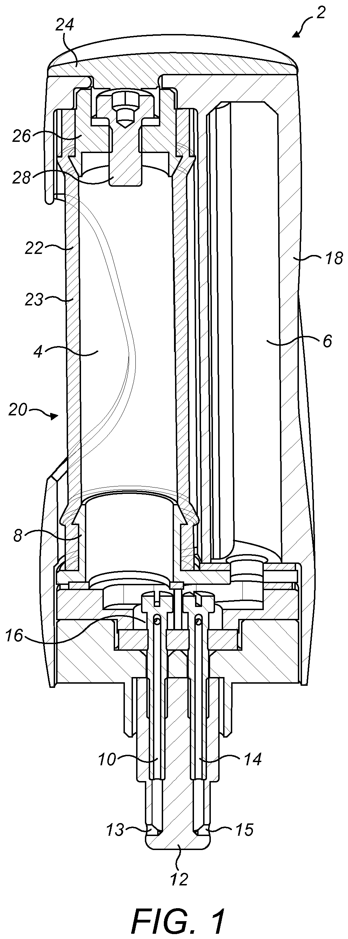

Figure 1 is a cross-sectional view of a refilling bottle 2. The bottle 2 comprises a supply reservoir 4 comprising an e-liquid which is an aerosol forming substance, otherwise referred to as an aerosol forming precursor. The bottle 2 also comprises a return reservoir 6. The supply reservoir 4 and return reservoir 6 are arranged in fluid communication with one another and are arranged with main axes that are substantially parallel along the length of the bottle 2. In alternative arrangements the supply reservoir 4 and return reservoir 6 may be provided with different sizes and shapes.

A hose connector 8 is provided at one end of the supply reservoir 4 for providing a fluid connection between the supply reservoir 4 and a supply conduit 10. The supply conduit 10 extends from the hose connector 8 and through a protrusion 12 that extends from one end of the bottle 2.

A return conduit 14 is provided in the protrusion 12 symmetrically with the supply conduit 10. The return conduit 14 extends from the return reservoir 6 through the protrusion 12.

The supply conduit 10 and return conduit 14 have an outlet 13 and an inlet 15 respectively, positioned towards one end of the protrusion 12. The outlet and inlet 13, 15 are arranged respectively to point in radial directions with respect to the main axis of the protrusion 12. More specifically, the outlet and inlet 13, 15 are arranged to point in mutually opposite directions that are separated from one another by around 180° with respect to the main axis of the protrusion 12.

A valve 16 is provided at the upper ends of the supply conduit 10 and return conduit 14. The valve 16 comprises a spring (not shown) that closes the supply conduit 10 and the return conduit 14 when the bottle 2 is not connected to an aerosol inhaler 30 such as an electronic cigarette.

A hard plastic housing 18 is provided around the periphery of the supply reservoir 4 and the return reservoir 6. A cut-out 20 in the hard plastic housing 18 is provided at a position along the length of the supply reservoir 4. The supply reservoir 4 is formed by a silicone hose 22 having at least one flexible wall 23. The silicone hose 22 is exposed in the region of the cut-out 20. In use, the flexible wall 23 of the supply reservoir 4 can be flexed inwardly by a user. The inward flexing of the wall 23 causes an increase in pressure in the supply reservoir 4 that can drive the aerosol forming substance through the supply conduit 10.

A removable housing cap 24 is provided at an opposite end of the bottle 2 to the protrusion 12. The removable housing cap 24 can be removed to reveal a refill stopper 26 at one end of the supply reservoir 4. The refill stopper 26 includes a threaded portion 28 that can be unscrewed. When the supply reservoir 4 has been depleted the removable housing cap 24 can be removed and the refill stopper 26 can be unscrewed. The supply reservoir 4 can then be refilled from another source.

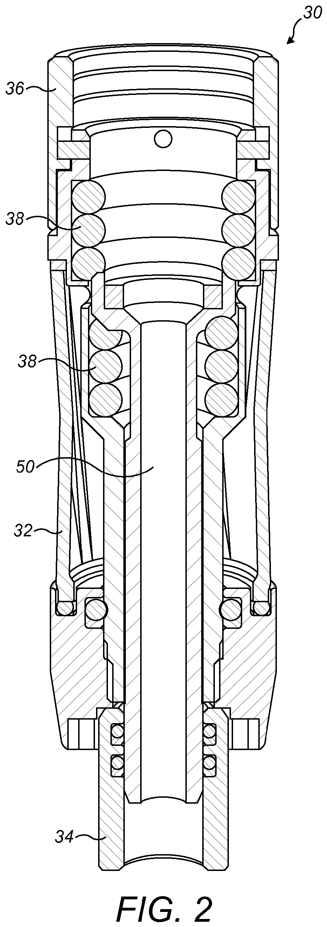

Figure 2 is a cross-sectional view of an aerosol inhaler 30 having a tank 32, otherwise referred to as a receiving reservoir, which can be refilled with the bottle 2. The aerosol inhaler 30 includes a mouthpiece 34 at one end and a cap nut 36 at the other end. The protrusion 12 of the bottle 2 is adapted to be inserted into the aerosol inhaler 30 at the position of the cap nut 36 so that it engages with a seal and spring o-ring package 38. The seal and spring o-ring package 38 is adapted to close access to the tank 32 when the bottle 2 is disconnected and to open access to the tank 32 for refilling when the bottle 2 is connected. This provides a hermetically sealed circuit which includes the supply reservoir 4, the supply conduit 10, the tank 32, the return conduit 14 and the return reservoir 6. A centre jacket 50 is provided, which is sealed from the tank 32. Thus, the tank 32 has an annular cross-sectional shape with the centre jacket 50 extending through the centre.

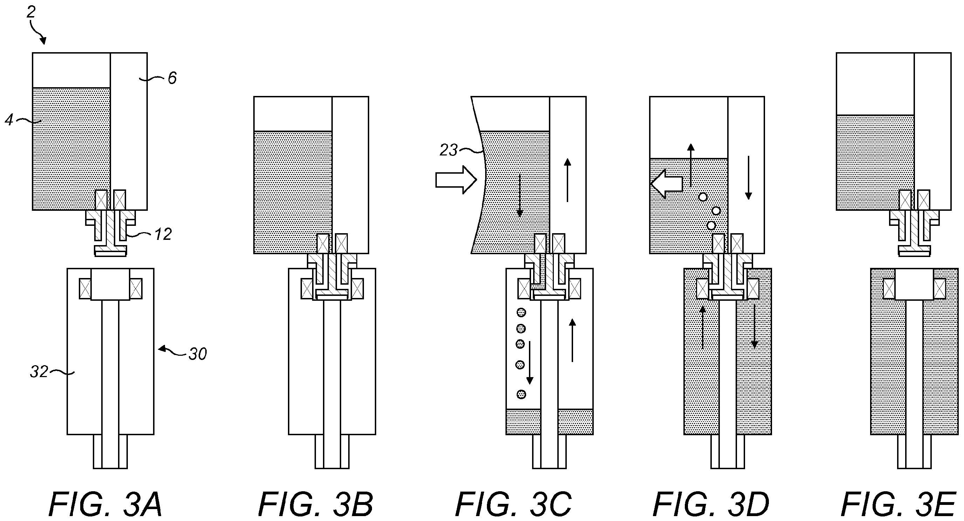

Figures 3A-3E show the steps that can be performed in a refilling operation. As shown in Figure 3A the refilling procedure begins when the tank 32 in the aerosol inhaler 30 has been depleted. A user then removes a battery (not shown) from the remainder of the aerosol inhaler 30. As shown in Figure 3B the bottle 2 is then connected to the aerosol inhaler 30. In this embodiment a threaded connection is provided between the bottle 2 and the aerosol inhaler 30. Of course, this is just one option and a variety of other connections could be used in alternatives, including plug and socket, press fit and bayonet. When the bottle 2 and the aerosol inhaler 30 are connected the protrusion 12 establishes a hermetic seal between the supply conduit 10, the tank 32 and the return conduit 14. As shown in Figure 3C, a user can squeeze the bottle 2 and displace the flexible silicone wall 23 of the supply reservoir 4 inwards. The inward displacement of the wall 23 causes an increase in fluid pressure in the supply reservoir 4 that urges aerosol forming substance into the supply conduit 10 and towards the tank 32. The aerosol forming substance is delivered to the tank 32 from the outlet 13 of the supply conduit 10. The delivery of the aerosol forming substance to the tank 32 displaces fluid that is already present in the tank 32, and this displaced fluid is received by the inlet 15 of the return conduit 14. The displaced fluid may include a gaseous component and a liquid component, but it will generally be constituted of gas. The displaced fluid is transmitted through the return conduit 14 to be received in the return reservoir 6.

When the user squeezes the bottle 2 the displacement of the silicone wall 23 causes a compression of fluid in the hermetically sealed circuit. The circuit will generally include at least some gaseous component, particularly because refilling is undertaken only when the tank 32 is depleted of aerosol forming substance. Thus, the displacement of the wall 23 means that the volume of gas in the sealed circuit is reduced. In fact, the volume of gas in the sealed circuit is reduced by the same amount as the volume of aerosol forming substance that is displaced by the flexible wall 23. When the user releases the flexible wall 23 the compressed gas in the sealed circuit typically expands and the volume is returned to its previous value. This provides a pneumatic return force on the flexible wall 23, sometimes referred to as an air spring, causing it to return to its original configuration.

The flexible wall 23 is elastomeric, and a mechanical return force acts on the wall 23 when it is displaced from its resting position. The combined effect of the mechanical return force and the pneumatic return force means that the flexible wall 23 tends to snap back to its original configuration after the user releases the squeezing force, as shown in Figure 3D. It has been found that this provides a desirable user experience.

The flexible wall 23 of the supply reservoir 4 is typically compressed by the user placing one or more fingers on the wall 23 and squeezing it inwards. The depth and size of the cut-out 20 generally influences the volume of the aerosol forming substance that is displaced from the supply reservoir 4 when the flexible silicone wall 23 is flexed inwards. The depth and size of the cut-out 20 is selected so that the volume of dispensed aerosol forming substance is approximately the same as the volume of the tank 32. In this way, the tank 32 can be refilled by a single depression of the flexible wall 23 by the user. In embodiments the volume of the tank 32 may be around 1ml. The volume of the supply reservoir 4 may be around 2ml to allow for around two refills before the bottle 2 must be refilled itself from another source. The volume of the return reservoir 6 may be around 2ml.

When the refilling operation is complete the bottle 2 is detached from the aerosol inhaler 30, as shown in Figure 3E. The seal and spring o-ring package 38 wipe the protrusion 12 as it is withdrawn to minimise any leakage of the aerosol forming substance outside of the sealed system. The battery (not shown) can then be replaced on the aerosol inhaler 30, ready for use.

In the arrangement above, the cut-out 20 and the flexible silicone wall 23 are shown as part of the supply reservoir 4. In an alternative arrangement these features could be provided as part of the return reservoir 6. This would provide a similar pumping arrangement for driving the aerosol forming substance from the supply reservoir 4 to the tank 32, via the supply conduit, and for allowing fluid displaced from the tank 32 to exit to the return reservoir 4, via the return conduit 14.

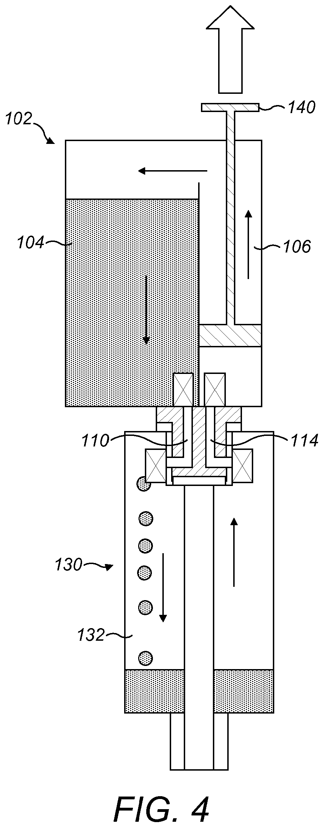

Figure 4 is a schematic view of another embodiment. In this arrangement the aerosol inhaler 130 is unchanged. However, a different mechanism is provided for displacing the aerosol forming substance from the bottle 102. In this arrangement a plunger 140 is provided in the return reservoir 106. The plunger 140 is shown in an initially extended position in the return reservoir 106. In use, the plunger 140 is retracted and fluid (generally a gas) is drawn from the tank 132 through the return conduit 114 to the return reservoir 106. In this way, a negative pressure is created in the tank 32 which, in turn, draws aerosol forming fluid into the tank 32 from the supply reservoir 104, through the supply conduit 110. The return reservoir 106 has a fluid connection with the supply reservoir 104 at their respective upper ends. Any liquid that is drawn into the return reservoir 106 can be poured into the supply reservoir 104 by tipping the bottle 2. The retraction of the plunger 140 dispenses a volume of aerosol forming substance from the supply reservoir 104 that is substantially matched to the volume of the tank 132.

Figure 4 shows the plunger 140 positioned in the return reservoir 106. A similar effect could be achieved in an alternative arrangement with the plunger 140 positioned in the supply reservoir 104.

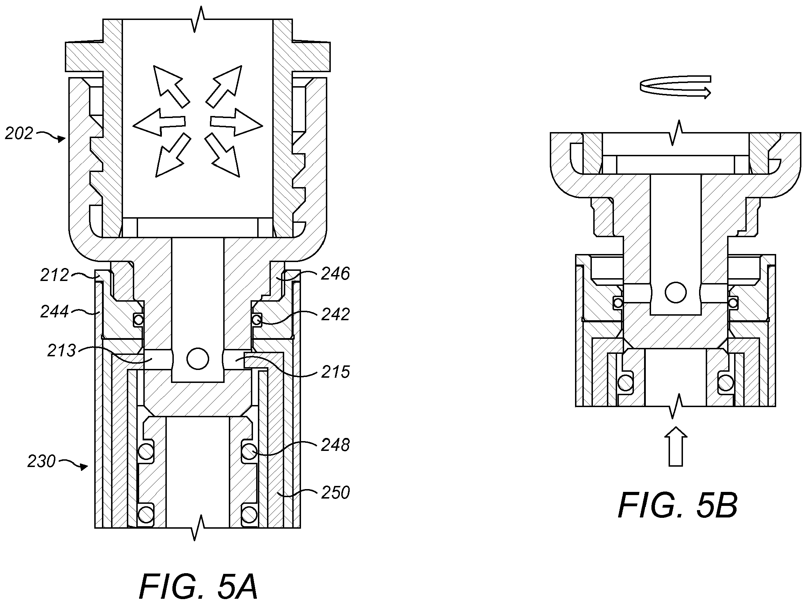

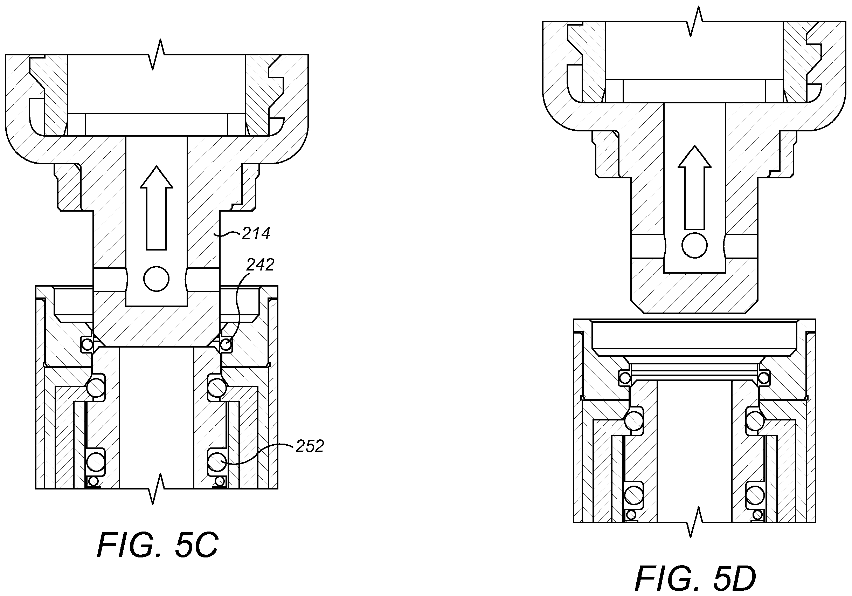

Figures 5A-5D are schematic cross-sectional views of a bottle 202 and aerosol inhaler 230 in an embodiment showing the steps that can be undertaken to detach the bottle 202 from the inhaler 230. The steps can simply be reversed for attachment of the bottle 202 to the inhaler 230. The bottle 202 comprises a protrusion 212 having an inlet 215 and outlet 213 of the supply conduit and return conduit respectively. As shown in Figure 5, when the bottle 202 is connected to the inhaler 230 the protrusion 212 is received in a receiving portion 246 at an upper end of the inhaler 230. The protrusion 212 engages at its lower end with the seal and spring o-ring package and the seal and o-ring package is moved downwardly relative to an outer housing 244 of the inhaler 230. In this position the inlet 215 and outlet 213 of the supply conduit and return conduit respectively are in fluid communication with the tank of the inhaler 230. A first o-ring 242 is provided in the receiving portion 246 of the inhaler 230 to engage with the protrusion 212 of the bottle 202. The first o-ring 242 seals against the protrusion 212 at a position that is displaced from the tank 232 and the inlet 215 and outlet 213 of the supply conduit and return conduit respectively. Thus, the first o-ring 242 provides a hermetic seal, in use, between the supply conduit, the tank and the return conduit.

A second o-ring 248 is provided in the seal and o-ring package. In use, the second o-ring 248 seals against a centre jacket 250 of the inhaler 230, which is not part of the tank. The tank has an annular cross-sectional shape in this arrangement and the second o-ring 248 provides an effective lower seal to isolate the centre jacket 250 from the tank.

The bottle 202 is removed from the inhaler 230 by unscrewing the threaded connection. As shown in Figure 5B the protrusion 212 is then withdrawn through the receiving portion 246 of the inhaler. The inlet 215 and outlet 213 are withdrawn so that they are no longer in fluid connection with the tank. The first o-ring 242 wipes the end of the protrusion 212 as it is withdrawn to remove any excess aerosol forming substance. As the protrusion 212 is withdrawn the seal and o-ring package, which is spring biased, rises axially in the inhaler 230 relative to the outer housing 244. As shown in Figure 5C the continued withdrawal of the protrusion 212 causes a third o-ring 252 in the seal and o-ring package to seal against an upper end of the tank in order to prevent leakage. The second o-ring 248 seals against the centre jacket 250 to isolate it from the tank. The bottle 202 is shown fully removed in Figure 5D.

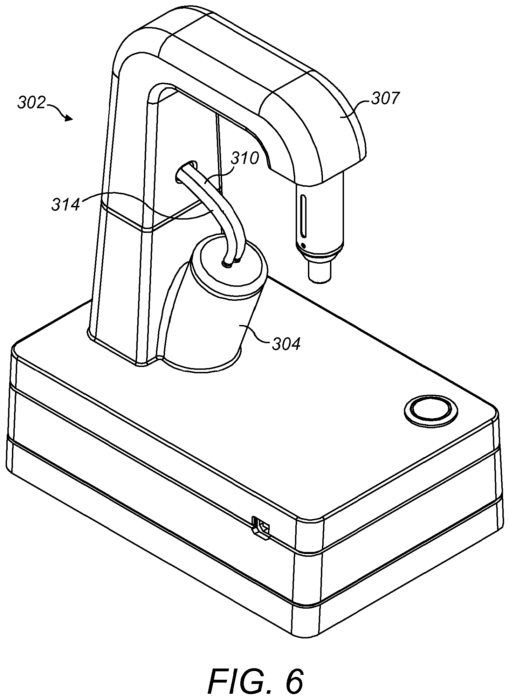

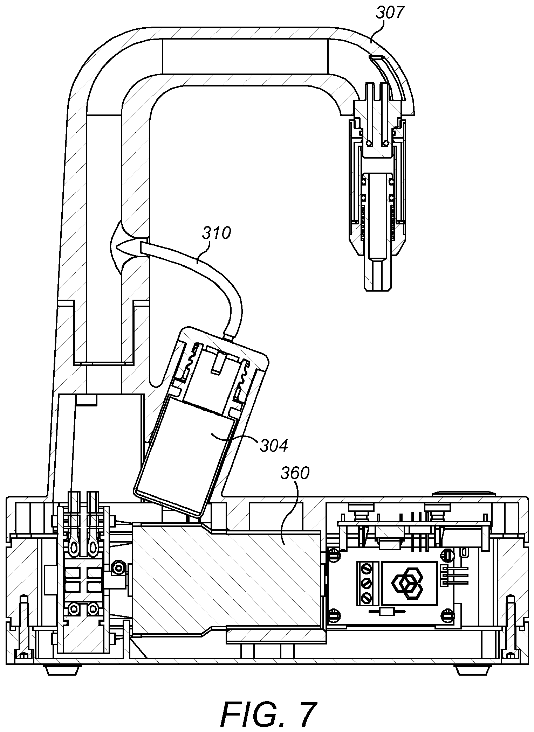

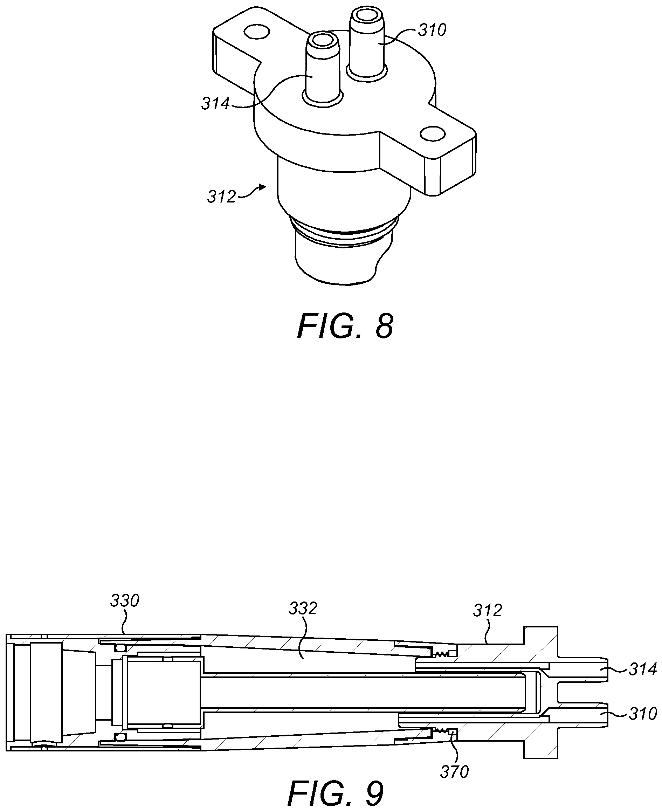

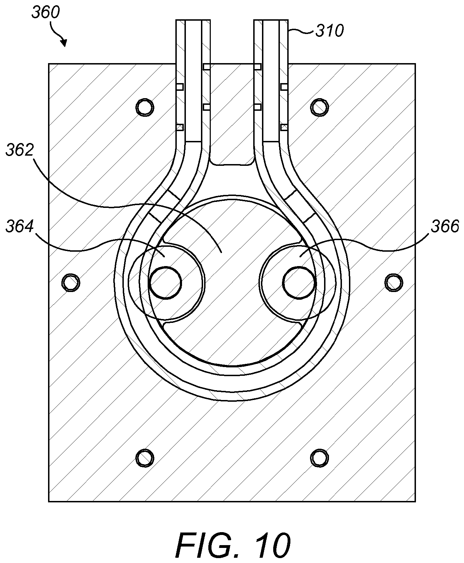

Another embodiment of the present invention is now described with reference to Figures 6 to 10. In this arrangement, a desktop refilling assembly 302 is provided including a supply reservoir 304 connected by a supply conduit 310 to a refill tap 307. A connector 312 is connectable at a distal end of the refill tap 307 for connecting the desktop refilling assembly 302 to an aerosol inhaler 330. The connector 312 comprises a supply conduit 310 operably connected to the supply reservoir 304 for providing a flow of aerosol forming substance from the supply reservoir 304 to a tank 332 in the aerosol inhaler 330. The connector 312 also includes a return conduit 314 configured to receive fluid displaced from the tank 332 when the aerosol forming substance is delivered and to supply the fluid to the supply reservoir 304. The supply conduit 310 and return conduit 314 are carried internally within the refill tap 307. In use, the connector 312 provides a hermetically sealed connection between the supply conduit 310, the tank 332 and the return conduit 314. An o-ring 370 is provided between the connector 312 and the aerosol inhaler 330 to establish a hermetically sealed connection.

A peristaltic pump 360 is provided for pumping the aerosol forming substance from the supply reservoir 304 towards the tank 332 through the supply conduit 310. In this embodiment the peristaltic pump 360 is electrically operated. However, it would be possible to provide a mechanically operated pump or a pneumatically operated pump in other embodiments. The peristaltic pump 360 comprises a rotor 362 and first and second rollers 364, 366. The supply conduit 310 is a flexible tube arranged in a u-bend around the rotor 362. In operation the rotor 362 is rotated and the first and second rollers 364, 366, which are displaceable members, provide moving constrictions in the supply conduit 310. In this way, the peristaltic pump 360 can provide a flow of the aerosol forming substance from the supply reservoir 304 in the supply conduit 310.

The peristaltic pump 360 comprises a 6V, 2.1W motor that can provide a flow rate of around 0.15ml/s. Advantageously the peristaltic pump 360 operates with a very low overpressure, which is close to zero. This compares favourably with hand operated pumps which can provide overpressures of perhaps around 1 bar. The low over pressure is achieved because the return conduit 314 transfers fluid out of the tank 332 at the same rate as the supply conduit 310 transfers the aerosol forming substance into the tank 332. In other words, the supply conduit 310 and the return conduit 314 provide move fluid in equal and opposite directions to and from the tank 332 (and to and from the supply reservoir 304).

It is desirable to provide the aerosol forming substance to the tank 332 with a low overpressure because the tank 332 typically has an outlet having a wicking material for providing the aerosol forming substance to an atomiser. The provision of a low pumping pressure means that in an embodiment wherein a cartomizer is refilled the aerosol forming substance is not urged out of the outlet of the tank 332 and into the wicking material, which could cause leakage. This system may be suitable for refilling a cartomiser (i.e. an open cartridge) without causing any undesirable leakage.

In an alternative arrangement the peristaltic pump 360 may be provided with the return conduit 314. This may provide effective pumping of fluid out of the tank 332, which creates a negative pressure drawing aerosol forming substance into the tank 332 through the supply conduit 310. Of course, separate peristaltic pumps 360 could be provided with both the supply conduit 310 and the return conduit 314.

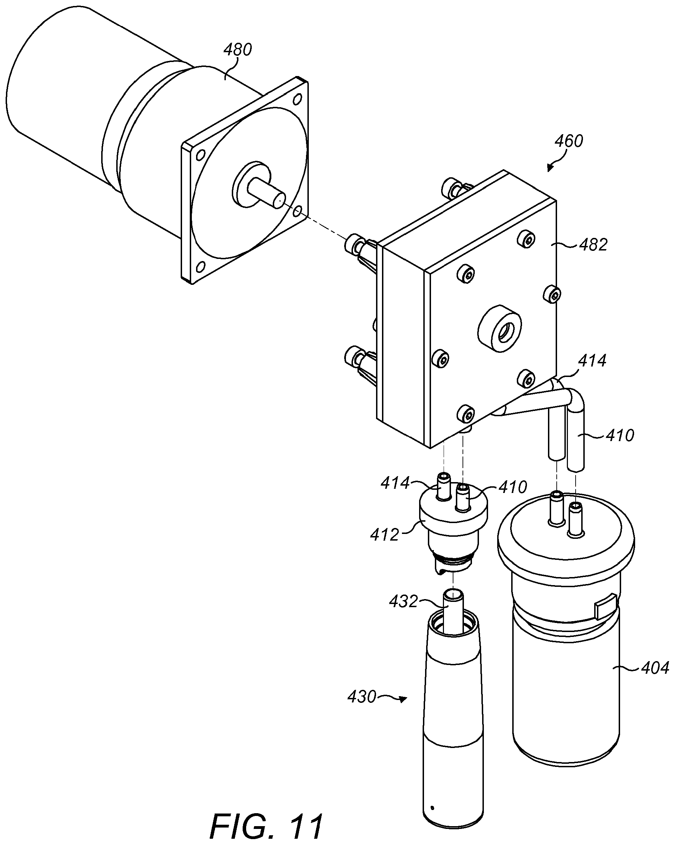

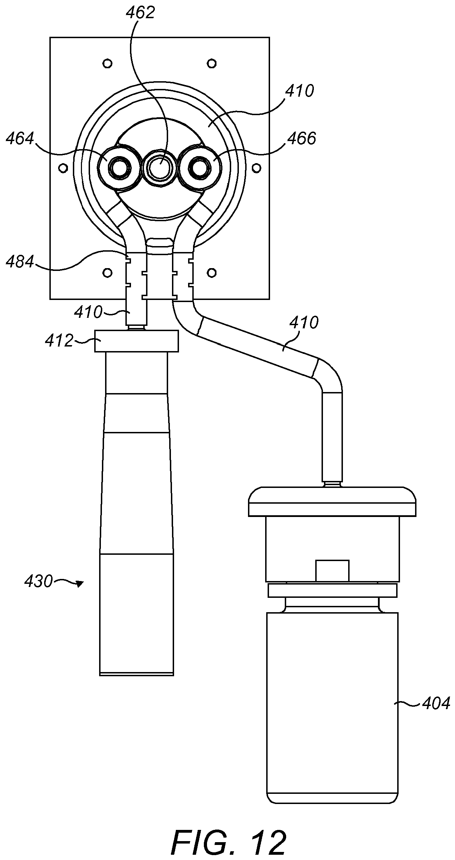

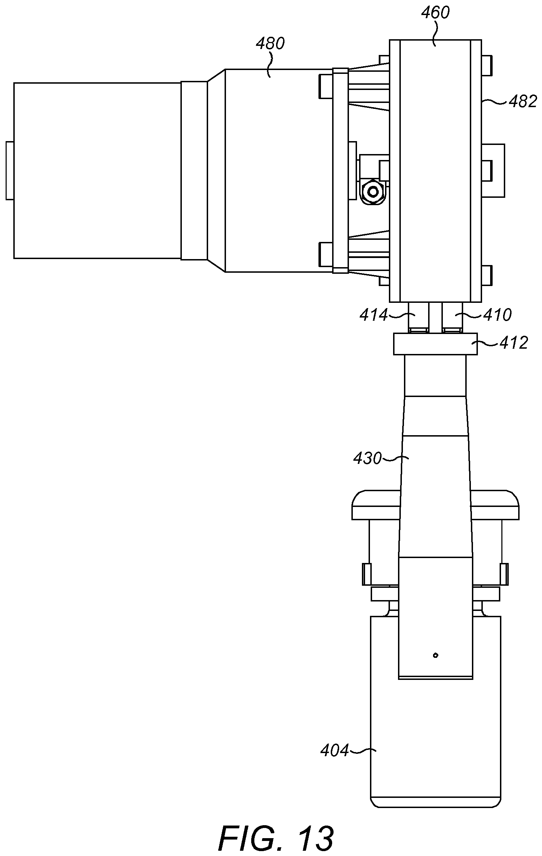

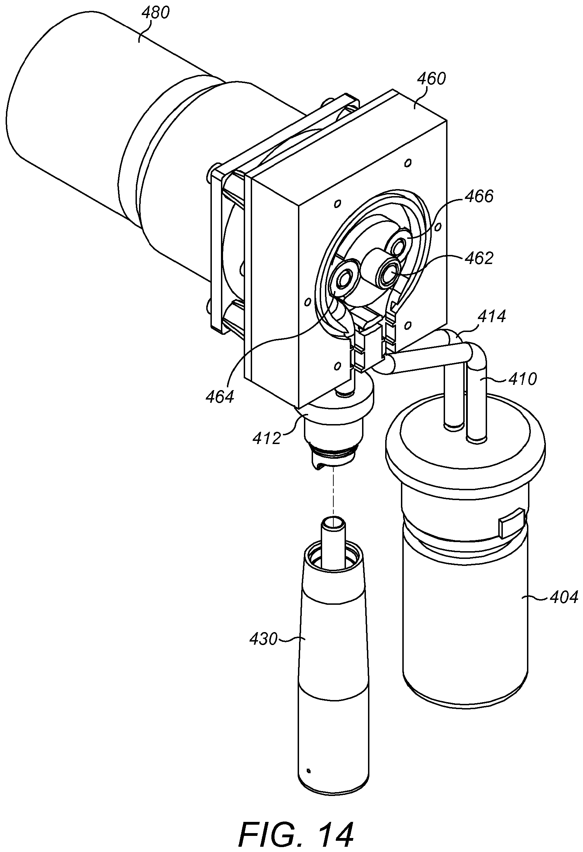

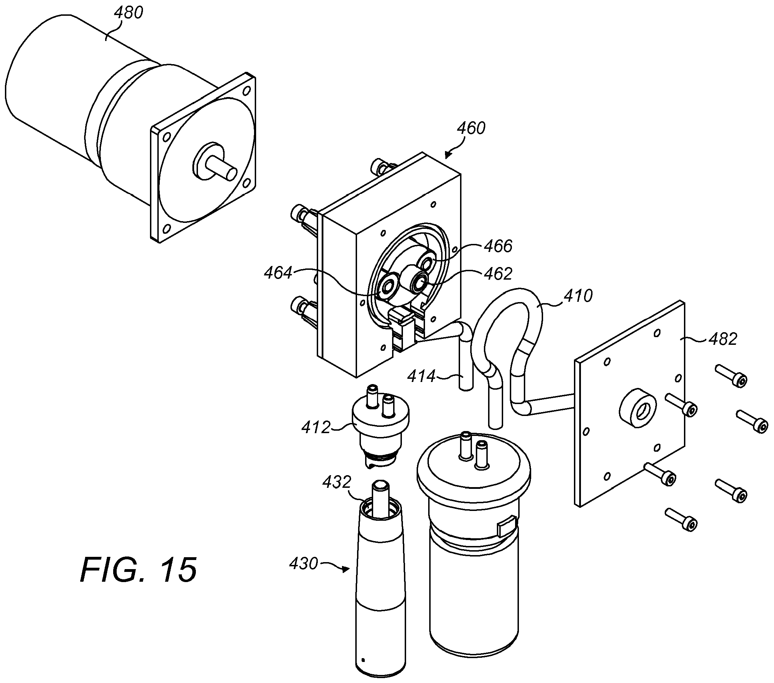

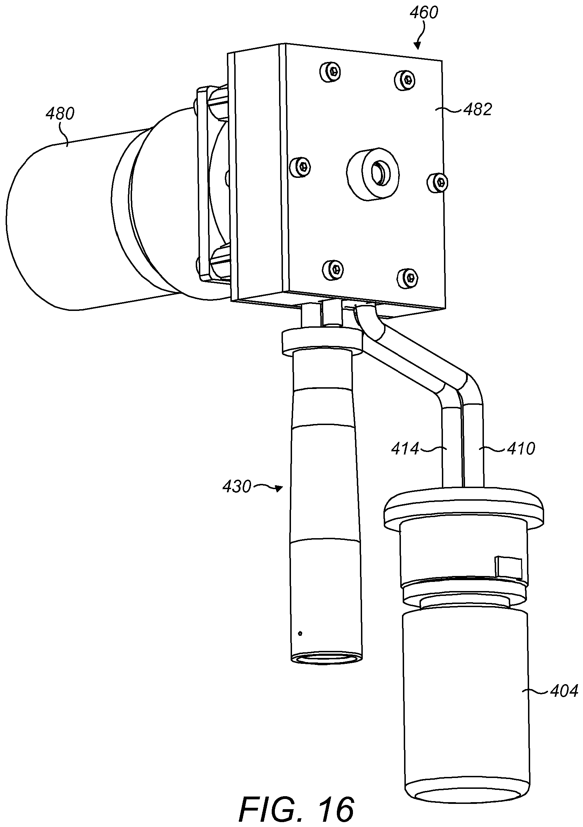

Another embodiment of the present invention is described with reference to Figures 11-16. In this arrangement an electric motor 480 is connected to a peristaltic pump 460. A cap 482 is provided on an outer casing of the peristaltic pump 460 to protect the operational elements, in use. A supply conduit 410 is provided between a supply reservoir 404 and a tank 432 in an aerosol inhaler 430. A return conduit 414 provides a return path between the tank 432 and the supply reservoir 404.

A connector 412 carries the supply conduit 410 and the return conduit 414 where they connect to the aerosol inhaler 430. In use, the connector 412 provides a hermetically sealed connection between the supply conduit 410, the tank 432 and the return conduit 414.

The peristaltic pump 460 comprises a rotor 462 and first and second rollers 464, 466 that provide moveable constrictions in the flexible supply conduit 410. In this arrangement only the supply conduit 410 is constricted by the rollers 464, 466 of the peristaltic pump 460. The return conduit 414 is not provided with a specific pump. In this arrangement the connector 412 is positioned close to the peristaltic pump 360. Specifically, the supply conduit 410 in the connector 412 is positioned adjacent an outlet 484 of the peristaltic pump 360. This advantageously minimises the volume of the aerosol forming substance that is held in the supply conduit 410 when the aerosol inhaler 430 is disconnected. This can help to reduce leakage when the aerosol inhaler 430 is disconnected. It has been found that the degree of constriction provided in the supply conduit 410 by the rollers 464, 466, the diameter of the supply conduit 410 and its length can have an impact on the amount of fluid leakage that can occur during or following disconnection.

Any reference numerals in the claims are not limiting on the scope of protection sought.

It will be understood that well known processes and elements have not been described in detail and may have been omitted for brevity. Specific steps, structures and materials have been described, by way of example. However, the present disclosure is not limited to those specific examples. It will be appreciated that some of the specific features described may be substituted for well-known alternatives, and that the method steps described may not necessarily be performed in the sequences given by way of example.

This disclosure has described a number of separate embodiments. However, it will be understood that features of different embodiments may be combined in any conceivable permutation. Other changes, substitutions, and alterations are also possible without departing from the scope of the claims.

## Claims

1. A refilling assembly (302) for refilling a receiving reservoir (332; 432) of an aerosol inhaler (330; 430) with an aerosol forming substance, the system comprising:
a supply reservoir (304; 404) to contain the aerosol forming substance;
a supply conduit (310; 410) in fluid communication with the supply reservoir for supplying the aerosol forming substance to the receiving reservoir;
a return conduit (314; 414) configured to receive fluid from the receiving reservoir when the aerosol forming substance is supplied to the receiving reservoir by the supply conduit;
a connection member (312; 412) configured to provide a sealed connection between the receiving reservoir, the supply conduit and the return conduit; and
a displaceable member (364, 366; 464, 466) operable to displace the aerosol forming substance from the supply reservoir, through the supply conduit, to the receiving reservoir, **characterised in that** the refilling assembly further comprises a peristaltic pump (360; 460) which includes the displaceable member, wherein the peristaltic pump is configured to pump the aerosol forming substance from the supply reservoir (104, 304) towards the receiving reservoir (332, 432).

2. The refilling assembly of claim 1, comprising a return reservoir (6, 106) fluid communication with the return conduit (314, 414), the return reservoir configured to receive fluid from the receiving reservoir (332, 432).

3. The refilling assembly of claim 2, wherein the supply reservoir (104, 304) and return reservoir (6, 106) are arranged in a common housing.

4. The refilling assembly of either of claims 2 or 3, wherein a sealed circuit is provided for transmission of fluid between the supply reservoir (104, 304) and the return reservoir (6, 106), via the receiving reservoir (332, 432), the sealed circuit including the supply reservoir (304, 404) the supply conduit (310, 410) the connection member (312, 412), and the return conduit (314, 414).

5. The refilling assembly of any of the preceding claims, wherein the peristaltic pump (360, 460) is an electric pump.

6. The refilling assembly of any of the preceding claims, wherein the displaceable member (364, 366, 464, 466) includes a first engagement member to provide a constriction to move along the supply conduit (310, 410) for pumping of the aerosol forming substance from the supply reservoir (304, 404) to the receiving reservoir (332, 432), and wherein the displaceable member includes a second engagement member to provide a constriction to move along the return conduit (314, 414) for pumping of the fluid from the supply reservoir to the receiving reservoir.

7. The refilling assembly of any of the preceding claims, wherein an outlet (13) of the supply conduit (310, 410) is arranged to point in a first direction and an inlet (15) of the return conduit (314, 414) is arranged to point in a second direction, wherein the first and second directions are different from one another.

8. The refilling assembly of claim 7, wherein the inlet (15) and outlet (13) are peripherally disposed about a common axis and the first and second directions are arranged on respective radially extending lines that are separated by 90 - 270° or 120 - 240°.

9. A system for refilling the receiving reservoir of an aerosol generation system with an aerosol forming substance, the system comprising:
the refilling assembly of any preceding claim; and
a storage portion including the receiving reservoir (332, 432) adapted to be connected to the connection member (312, 412).

10. Use of the refilling assembly of any of claims 1 to 8 for refilling a receiving reservoir (332, 432) of an aerosol generation system with an aerosol forming substance.

11. A method of refilling a receiving reservoir of an aerosol generation system with an aerosol forming substance, the method comprising the steps of:
providing a sealed connection between the receiving reservoir (332, 432), a supply conduit (310, 410) and a return conduit (314, 414);
actuating a displaceable member to displace the aerosol forming substance from the supply reservoir, through the supply conduit, to the receiving reservoir (332, 432); and
receiving fluid from the receiving reservoir in the return conduit;
**characterised in that** the method further comprises operating a peristaltic pump (360, 460)
which includes the displaceable member, wherein the peristaltic pump is configured to pump the aerosol forming substance from the supply reservoir (104, 304) towards the receiving reservoir.

## Patentansprüche

1. Nachfüllanordnung (302) zum Nachfüllen eines Empfangsbehälters (332; 432) eines Aerosol-Inhalators (330; 430) mit einer ein Aerosol bildenden Substanz, wobei das System umfasst:
Einen Vorratsbehälter (304; 404), um die Aerosol bildende Substanz zu beinhalten;
eine Versorgungsleitung (310; 410) in Flüssigkeitsverbindung mit dem Vorratsbehälter zur Lieferung der Aerosol bildenden Substanz an den Empfangsbehälter;
eine Rückleitung (314; 414), die ausgelegt ist, Flüssigkeit aus dem Empfangsbehälter zu empfangen, wenn die Aerosol bildende Substanz von der Versorgungsleitung zum Empfangsbehälter geliefert wird;
ein Verbindungselement (312; 412), das ausgelegt ist, eine abgedichtete Verbindung zwischen dem Empfangsbehälter, der Versorgungsleitung und der Rückleitung bereitzustellen; und
ein verschiebbares Element (364, 366; 464, 466), das betreibbar ist, die Aerosol bildende Substanz aus dem Vorratsbehälter, durch die Versorgungsleitung, zum Empfangsbehälter zu verlagern, **dadurch gekennzeichnet, dass** die Nachfüllanordnung ferner eine peristaltische Pumpe (360; 460) umfasst, die das verschiebbare Element einschließt, wobei die peristaltische Pumpe ausgelegt ist, die Aerosol bildende Substanz aus dem Vorratsbehälter (104, 304) in Richtung des Empfangsbehälters (332, 432) zu pumpen.

2. Nachfüllanordnung nach Anspruch 1, die einen Rücklaufbehälter (6, 106) in Flüssigkeitsverbindung mit der Rückleitung (314, 414) umfasst, wobei der Rücklaufbehälter ausgelegt ist, Flüssigkeit aus dem Empfangsbehälter (332, 432) zu empfangen.

3. Nachfüllanordnung nach Anspruch 2, wobei der Vorratsbehälter (104, 304) und der Rücklaufbehälter (6, 106) in einem gemeinsamen Gehäuse angeordnet sind.

4. Nachfüllanordnung entweder nach Ansprüchen 2 oder 3, wobei ein abgedichteter Kreislauf zur Übertragung von Flüssigkeit zwischen dem Vorratsbehälter (104, 304) und dem Rücklaufbehälter (6, 106), über den Empfangsbehälter (332, 432), bereitgestellt ist, wobei der abgedichtete Kreislauf den Vorratsbehälter (304, 404), die Versorgungsleitung (310, 410), das Verbindungselement (312, 412) und die Rückleitung (314, 414) einschließt.

5. Nachfüllanordnung nach irgendeinem der vorhergehenden Ansprüche, wobei die peristaltische Pumpe (360, 460) eine elektrische Pumpe ist.

6. Nachfüllanordnung nach irgendeinem der vorhergehenden Ansprüche, wobei das verschiebbare Element (364, 366, 464, 466) ein erstes Eingriffselement einschließt, um eine Verengung vorzusehen, um sich entlang der Versorgungsleitung (310, 410) zu bewegen, um die Aerosol bildende Substanz aus dem Vorratsbehälter (304, 404) zum Empfangsbehälter (332, 432) zu pumpen, und
wobei das verschiebbare Element ein zweites Eingriffselement einschließt, um eine Verengung vorzusehen, um sich entlang der Rückleitung (314, 414) zu bewegen, um die Flüssigkeit aus dem Vorratsbehälter zum Empfangsbehälter zu pumpen.

7. Nachfüllanordnung nach irgendeinem der vorhergehenden Ansprüche, wobei ein Auslass (13) der Versorgungsleitung (310, 410) angeordnet ist, in eine erste Richtung zu zeigen und ein Einlass (15) der Versorgungsleitung (314, 414) angeordnet ist, in eine zweite Richtung zu zeigen, wobei die ersten und zweiten Richtungen voneinander verschieden sind.

8. Nachfüllanordnung nach Anspruch 7, wobei der Einlass (15) und der Auslass (13) peripher um eine gemeinsame Achse angeordnet sind und die ersten und zweiten Richtungen auf jeweiligen, sich radial erstreckenden Linien angeordnet sind, die durch 90 - 270° oder 120 - 240° getrennt sind.

9. System zum Nachfüllen des Empfangsbehälters eines Aerosol-Erzeugungssystems mit einer Aerosol bildenden Substanz, wobei das System umfasst:
Die Nachfüllanordnung irgendeines vorhergehenden Anspruchs; und
einen den Empfangsbehälter (332, 432) einschließenden Speicherteil, der angepasst ist, an das Verbindungselement (312, 412) angeschlossen zu werden.

10. Verwenden der Nachfüllanordnung irgendeines der Ansprüche 1 bis 8 zum Nachfüllen eines Empfangsbehälters (332, 432) eines Aerosol-Erzeugungssystems mit einer Aerosol bildenden Substanz.

11. Verfahren zum Nachfüllen eines Empfangsbehälters eines Aerosol-Erzeugungssystems mit einer Aerosol bildenden Substanz, wobei das Verfahren folgende Schritte umfasst:
Bereitstellen einer abgedichteten Verbindung zwischen dem Empfangsbehälter (332, 432), einer Versorgungsleitung (310, 410) und einer Rückleitung (314, 414);
Betätigen eines verschiebbaren Elements, um die Aerosol bildende Substanz aus dem Vorratsbehälter, durch die Versorgungsleitung, zum Empfangsbehälter (332, 432) zu verlagern; und
Empfangen von Flüssigkeit aus dem Empfangsbehälter in die Rückleitung;
**dadurch gekennzeichnet, dass** das Verfahren ferner das Betreiben einer peristaltischen Pumpe (360, 460) umfasst, die das verschiebbare Element einschließt, wobei die peristaltische Pumpe ausgelegt ist, die Aerosol bildende Substanz aus dem Vorratsbehälter (104, 304) in Richtung des Empfangsbehälters zu pumpen.

## Revendications

1. Un ensemble de recharge (302) pour assurer le remplissage d'un réservoir de réception (332, 432) d'un inhalateur aérosol (330, 430) en faisant appel à une substance de formation d'aérosol et ce système se compose des éléments suivants :
un réservoir d'alimentation (304, 404) conçu pour contenir la substance de formation d'aérosol
une conduite d'alimentation (310, 410) en communication liquide avec le réservoir d'alimentation pour acheminer la substance de formation d'aérosol jusqu'au réservoir de réception
une conduite de retour (314, 414) configurée pour recevoir un liquide en provenance du réservoir de réception lorsque la substance de formation d'aérosol est acheminée jusqu'au réservoir de réception par le biais de la conduite d'alimentation
un élément de raccordement (312, 412) configuré pour assurer une connexion hermétiquement scellée entre le réservoir de réception, la conduite d'alimentation et la conduite de retour et
un élément de déplacement (364, 366, 464, 466) qui peut être utilisé pour assurer l'acheminement de la substance de formation d'aérosol depuis le réservoir d'alimentation, tout au long de la conduite d'alimentation et jusqu'au réservoir de réception et **se caractérisant par le fait que** cet ensemble de recharge comporte, en outre, une pompe péristaltique (360, 460), dont fait partie l'élément de déplacement, et cette pompe péristaltique est configurée pour assurer le pompage de la substance de formation d'aérosol depuis le réservoir d'alimentation (104, 304) et à destination du réservoir de réception (332, 432).

2. L'ensemble de recharge que décrit la revendication 1, si ce n'est qu'il comporte un réservoir de retour (6, 106) en communication liquide avec la conduite de retour (314, 414), et ce réservoir de retour est configuré pour recevoir le liquide en provenance du réservoir de réception (332, 432).

3. L'ensemble de recharge que décrit la revendication 2, si ce n'est que le réservoir d'alimentation (104, 304) et le réservoir de retour (6, 106) sont implantés dans un même coffret.

4. L'ensemble de recharge que décrit la revendication 2 ou la revendication 3, si ce n'est qu'un circuit hermétiquement scellé est prévu pour assurer la transmission de liquide entre le réservoir d'alimentation (104, 304) et le réservoir de retour (6, 106), via le réservoir de réception (332, 432), et ce circuit hermétiquement scellé se compose du réservoir d'alimentation (304, 404), de la conduite d'alimentation (310, 410), de l'élément de raccordement (312, 412) et de la conduite de retour (314, 414).

5. L'ensemble de recharge que décrit l'une ou l'autre des revendications précédentes, si ce n'est que la pompe péristaltique (360, 460) est une pompe électrique.

6. L'ensemble de recharge que décrit l'une ou l'autre des revendications précédentes, si ce n'est que l'élément de déplacement (364, 366, 464, 466) comporte un premier élément de mise en prise qui fournit un étranglement permettant le déplacement le long de la conduite d'alimentation (310, 410) afin de pomper la substance de formation de l'aérosol depuis le réservoir d'alimentation (304, 404) et jusqu'au réservoir de réception (332, 432), et si ce n'est que cet élément de déplacement comporte un deuxième élément de mise en prise qui fournit un étranglement permettant le déplacement le long de la conduite de retour (314, 414) afin de pomper le liquide depuis le réservoir d'alimentation, jusqu'au réservoir de réception.

7. L'ensemble de recharge que décrit l'une ou l'autre des revendications précédentes, si ce n'est qu'une sortie (13) de la conduite d'alimentation (310, 410) est positionnée de manière à pointer dans un premier sens et qu'une entrée (15) de la conduite de retour (314, 414) est positionnée de manière ä pointer dans un deuxième sens, et ce premier sens est différent du deuxième sens.

8. L'ensemble de recharge que décrit la revendication 7, si ce n'est que l'entrée (15) et la sortie (13) sont implantées sur le pourtour et autour d'un axe commun et que le premier sens et le deuxième sens sont implantés sur des lignes respectives, sur le plan radial, qui ont une séparation angulaire de 90-270° ou 120-240°.

9. Un système de recharge du réservoir de réception d'un système de production d'aérosol à l'aide d'une substance de formation d'aérosol, et ce système se compose des éléments suivants :
l'ensemble de recharge que décrit l'une ou l'autre des revendications précédentes et
une section de stockage qui se compose du réservoir de réception (332, 432) spécialement adapté pour se brancher sur l'élément de raccordement (312, 412).

10. L'utilisation de l"ensemble de recharge que décrit l'une ou l'autre des revendications 1 à 8 pour assurer la recharge d'un réservoir de réception (332, 432) d'un système de production d'un aérosol à l'aide d'une substance de formation d'aérosol.

11. Un procédé de recharge d'un réservoir de réception à l'aide d'un système de production d'aérosol, et ce procédé se compose des éléments suivants :
fourniture d'une connexion hermétiquement scellée entre le réservoir de réception (332, 432), une conduite d'alimentation (310, 410) et une conduite de retour (314, 414)
actionnement d'un élément de déplacement pour acheminer la substance de formation d'aérosol depuis le réservoir d'alimentation, la faire traverser la conduite d'alimentation et l'amener au réservoir de réception (332, 432) et
réception du fluide en provenance du réservoir de réception via la conduite de retour
**se caractérisant par le fait que** ce procédé comporte, en outre, le fonctionnement d'une pompe péristaltique (360, 460)
qui contient l'élément de déplacement et cette pompe péristaltique est configurée pour pomper la substance de formation d'aérosol depuis le réservoir d'alimentation (104, 304) et en direction du réservoir de réception.
